(19) Europäisches Patentamt
European Patent Office
Office européen des brevets

(11) Veröffentlichungsnummer: **0 008 331**
**B1**

(12) # EUROPÄISCHE PATENTSCHRIFT

(45) Veröffentlichungstag der Patentschrift: **20.01.82**

(51) Int. Cl.³: **C 07 C 69/743,** A 01 N 53/00, C 07 C 43/20

(21) Anmeldenummer: **79102084.5**

(22) Anmeldetag: **25.06.79**

(54) **Cyclopropancarbonsäureester, ihre Herstellung und Verwendung als Schädlingsbekämpfungsmittel.**

(30) Priorität: **27.06.78 CH 6990/78**
**19.03.79 CH 2558/79**

(43) Veröffentlichungstag der Anmeldung: **05.03.80 Patentblatt 80/5**

(45) Bekanntmachung des Hinweises auf die Patenterteilung: **20.01.82 Patentblatt 82/3**

(84) Benannte Vertragsstaaten: **AT BE CH DE FR GB IT NL**

(56) Entgegenhaltungen:
**GB-A-1 467 579**

(73) Patentinhaber: **CIBA-GEIGY AG, Patentabteilung Postfach, CH-4002 Basel (CH)**

(72) Erfinder: **Farooq, Saleem, Dr., Im Schalengarten 2, CH-4107 Ettingen (CH)**
Erfinder: **Ackermann, Peter, Dr., Reichensteinerstrasse 12, CH-4153 Reinach (CH)**
Erfinder: **Drabek, Jozef, Dr., Benkenstrasse 12, CH-4104 Oberwil (CH)**
Erfinder: **Gsell, Laurenz, Dr., Maiengasse 56, CH-4056 Basel (CH)**
Erfinder: **Kristiansen, Odd, Dr., Delligrabenstrasse 7, CH-4313 Möhlin (CH)**
Erfinder: **Wehrli, Rudolf, Dr., Dianastrasse 2, CH-4310 Rheinfelden (CH)**

(74) Vertreter: **Zumstein sen., Fritz, Dr. et al, Bräuhausstrasse 4, D-8000 München 2 (DE)**

EP 0 008 331 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Cyclopropancarbonsäureester, ihre Herstellung und Verwendung als Schädlingsbekämpfungsmittel

Die vorliegende Erfindung betrifft Cyclopropancarbonsäureester, Verfahren zu ihrer Herstellung und ihre Verwendung in der Schädlingsbekämpfung.

Die Cyclopropancarbonsäureester haben die Formel

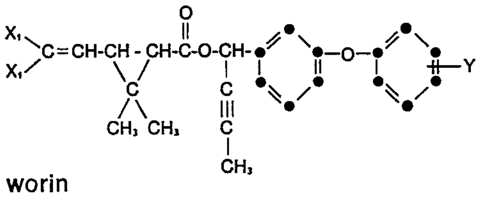

(I)

worin

$X_1$ Fluor, Chlor oder Brom und

Y Methoxy, Methyl, Fluor, Chlor oder Brom bedeuten.

Wegen ihrer Wirkung von besonderer Bedeutung sind Verbindungen der Formel I, worin $X_1$ Fluor, Chlor oder Brom und Y p-Methoxy, p-Methyl, p-Fluor, p-Chlor oder p-Brom bedeuten.

Die anmeldungsgemässen Verbindungen weisen gegenüber den aus der GB-PS 1 438 129 und GB-PS 1 467 579 bekannten, strukturell nahe verwandten Produkten einen überraschend geringeren Toxizitätslevel auf.

Die Verbindungen der Formel I werden nach an sich bekannten Methoden z.B. wie folgt hergestellt:

1)

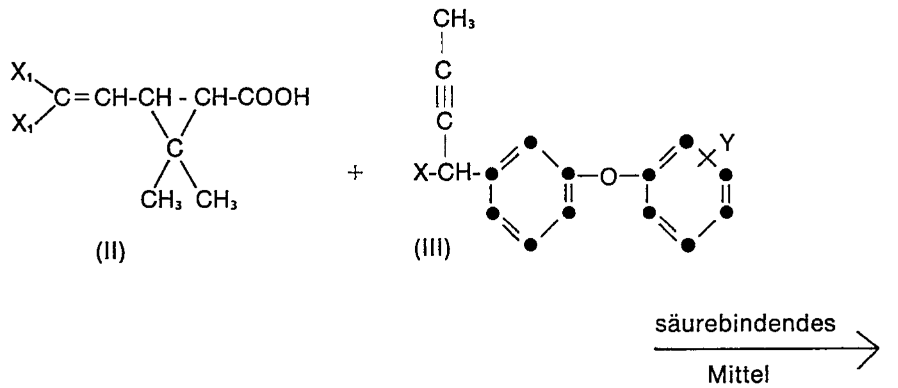


(II) (III)

2)

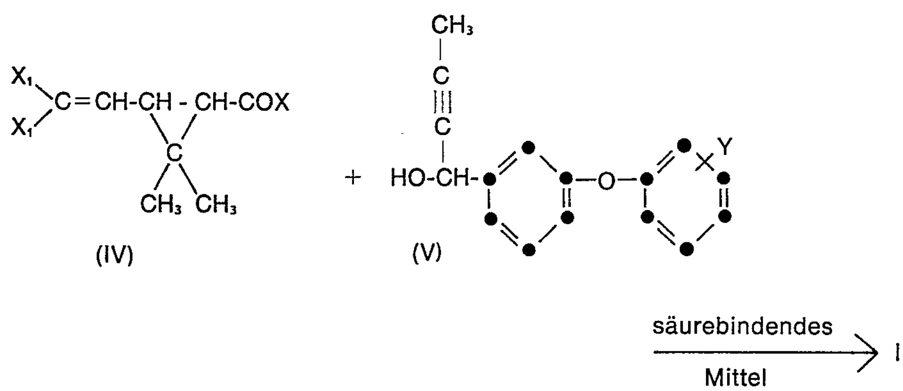


(IV) (V)

3)

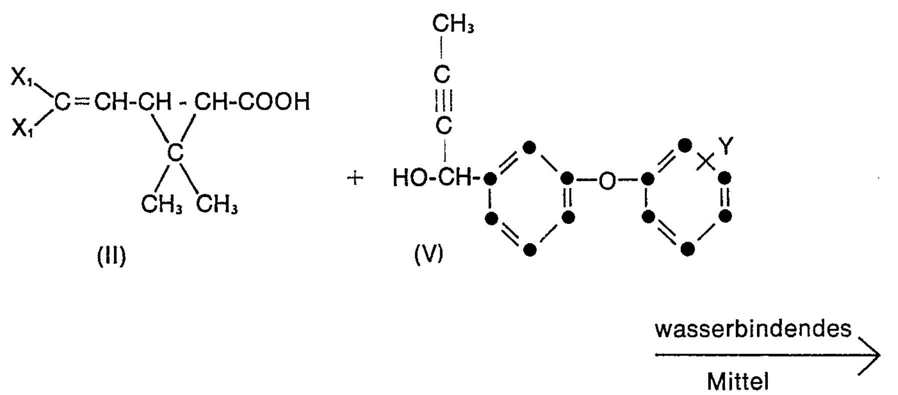


(II) (V)

4)

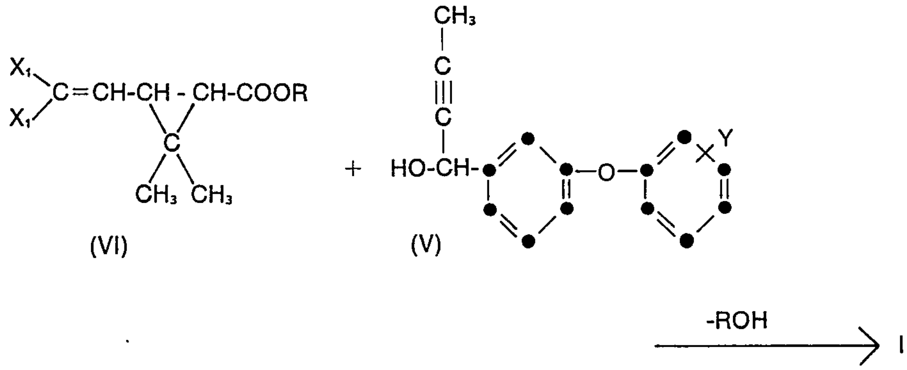


(VI) (V)

In den Formeln II bis VI haben $X_1$ und Y die für die Formel I angegebene Bedeutung.

In den Formeln III und IV steht X für ein Halogenatom, insbesondere Chlor oder Brom, und in der Formel VI steht R für $C_1$-$C_4$-Alkyl, insbesondere für Methyl oder Äthyl. Als säurebindendes Mittel für die Verfahren 1 und 2 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate, wie z.B. Kalium-t.butylat und Natriummethylat in Betracht. Als wasserbindendes Mittel für das Verfahren 3 kann z.B. Dicyclohexylcarbodiimid verwendet werden. Die Verfahren 1 bis 4 werden bei einer Reaktionstemperatur zwischen −10 und 120°C, meist zwischen 20 und 80°C bei normalem oder erhöhtem Druck und vorzugsweise in einem inerten Lösungs- oder Verdünnungsmittel durchgeführt. Als Lösungs- oder Verdünnungsmittel eignen sich z.B. Äther und ätherartige Verbindungen, wie Diäthyläther, Dipropyläther, Dioxan, Dimethoxyäthan und Tetrahydrofuran; Amide, wie N,N-dialkylierte Carbonsäureamide; aliphatische, aromatische sowie halogenierte Kohlenwasserstoffe, insbesondere Benzol, Toluol, Xylole, Chloroform und Chlorbenzol; Nitrile wie Acetonitril; Dimethylsulfoxid und Ketone wie Aceton und Methyläthylketon.

Die Ausgangsstoffe der Formeln II bis VI sind bekannt oder können analog bekannten Methoden hergestellt werden.

Die Verbindungen der Formel I liegen als Gemisch von verschiedenen optisch aktiven Isomeren vor, wenn bei der Herstellung nicht einheitlich optisch aktive Ausgangsmaterialien verwendet wurden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen tierischen und pflanzlichen Schädlingen.

Insbesondere eignen sich die Verbindungen der Formel I zur Bekämpfung von Insekten, phytopathogenen Milben und von Zecken z.B. der Ordnungen Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. gegen Spodoptera littoralis und Heliothis virescens) und Gemüsekulturen (z.B. gegen Leptinotarsa decemlineata und Myzus persicae).

Wirkstoffe der Formel I zeigen auch eine sehr günstige Wirkung gegen Fliegen, wie z.B. Musca domestica und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-[2-(octylsulfinyl)-propyl]-benzol.

Verbindungen der Formel I können für sich allein oder zusammen mit geeigneten Trägern und/oder Zuschlagstoffen eingesetzt werden. Geeignete Träger- und Zuschlagstoffe können fest oder flüssig sein und entsprechend den in der Formulierungstechnik üblichen Stoffen, wie z.B. natürlichen oder regenerierten Stoffen, Lösungs-, Dispergier-, Netz-, Haft-, Verdickungs-, Binde- und/oder Düngemitteln.

Die Herstellung erfindungsgemässer Mittel erfolgt in an sich bekannter Weise durch inniges Vermischen und/oder Vermahlen der Wirkstoffe der Formel I mit den geeigneten Trägerstoffen, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden:

Feste Aufarbeitungsformen:

Stäubemittel, Streumittel, Granulate (Umhüllungsgranulate, Imprägnierungsgranulate und Homogranulate);

Flüssige Aufarbeitungsformen:

a) in Wasser dispergierbare Wirkstoffkonzentrate: Spritzpulver (wettable powders), Pasten, Emulsionen;

b) Lösungen.

Der Gehalt an Wirkstoff in den oben beschriebenen Mitteln liegt zwischen 0,1 bis 95%, dabei ist zu erwähnen, dass bei der Applikation aus dem Flugzeug oder mittels anderer geeigneter Applikationsgeräte Konzentrationen bis zu 99,5 % oder sogar reiner Wirkstoff eingesetzt werden können. Die Wirkstoffe der Formel I können beispielsweise wie folgt formuliert werden (Teile bedeuten Gewichtsteile):

Stäubemittel:

Zur Herstellung eines a) 5%igen und b) 2%igen Stäubemittels werden die folgenden Stoffe verwendet:

a) 5 Teile Wirkstoff
95 Teile Talkum;

b) 2 Teile Wirkstoff
1 Teil hochdisperse Kieselsäure
97 Teile Talkum.

Der Wirkstoff wird mit den Trägerstoffen vermischt und vermahlen.

Granulat:

Zur Herstellung eines 5%igen Granulates werden die folgenden Stoffe verwendet:

5 Teile Wirkstoff
0,25 Teile Epichlorhydrin
0,25 Teile Cetylpolyglykoläther
3,50 Teile Polyäthylenglykol
91 Teile Kaolin (Korngrösse 0,3-0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver:

Zur Herstellung eines a) 40%igen, b) und c) 25%igen, d) 10%igen Spritzpulvers werden folgende Bestandteile verwendet:

a) 40 Teile Wirkstoff
5 Teile Ligninsulfonsäure-Natriumsalz
1 Teil Dibutylnaphthalinsulfonsäure-Natriumsalz
54 Teile Kieselsäure;

b) 25 Teile Wirkstoff
4,5 Teile Calcium-Ligninsulfonat
1,9 Teile Champagne-Kreide/Hydroxyäthylcellulose-Gemisch (1 : 1)
1,5 Teile Natrium-dibutyl-naphthalinsulfonat
19,5 Teile Kieselsäure
19,5 Teile Champagne-Kreide
28,1 Teile Kaolin;

c) 25 Teile Wirkstoff
2,5 Teile Isooctylphenoxy-polyäthylenäthanol
1,7 Teile Champagne-Kreide/Hydroxyäthylcellulose-Gemisch (1 : 1)
8,3 Teile Natriumaluminiumsilikat
16,5 Teile Kieselgur
46 Teile Kaolin;

d) 10 Teile Wirkstoff
3 Teile Gemisch der Natriumsalze von gesättigten Fettalkoholsulfaten
5 Teile Naphthalinsulfonsäure/Formaldehyd-Kondensat
82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate:

Zur Herstellung eines a) 10%igen, b) 25%igen und c) 50%igen emulgierbaren Konzentrates werden folgende Stoffe verwendet:

a) 10 Teile Wirkstoff
3,4 Teile epoxydiertes Pflanzenöl
3,4 Teile eines Kombinationsemulgators, bestehend aus Fettalkoholpolyglykoläther und Alkylarylsulfonat-Calcium-Salz
40 Teile Dimethylformamid
43,2 Teile Xylol;

b) 25 Teile Wirkstoff
2,5 Teile epoxydiertes Pflanzenöl
10 Teile eines Alkylarylsulfonat/Fettalkoholpolyglykoläther-Gemisches
5 Teile Dimethylformamid
57,5 Teile Xylol;

c) 50 Teile Wirkstoff
4,2 Teile Tributylphenol-Polyglykoläther
5,8 Teile Calcium-Dodecylbenzolsulfonat
20 Teile Cyclohexanon
20 Teile Xylol.

Aus solchen Konzentrationen können durch Verdünnen mit Wasser Emulsionen jeder gewünschten Konzentration hergestellt werden.

Sprühmittel:

Zur Herstellung eines a) 5%igen und b) 95%igen Sprühmittels werden die folgenden Bestandteile verwendet:

a) 5 Teile Wirkstoff
1 Teil Epichlorhydrin
94 Teile Benzin (Siedegrenzen 160-190°C);

b) 95 Teile Wirkstoff
5 Teile Epichlorhydrin.

Beispiel 1

a) Herstellung von α-Prop-1-ynyl-3-p-chlorphenoxybenzylalkohol

Zu einer Lösung von 8 g Methylacetylen in 100 ml Tetrahydrofuran wird bei 0°C eine frisch aus 4 g Magnesium, 20 g Äthylbromid in 20 ml Tetrahydrofuran hergestellte Grignardlösung

langsam zugegeben und während 15 Minuten unter Argon gerührt. Zu diesem Gemisch wird eine Lösung von 33,6 g o-p-Chlorphenoxybenzaldehyd in 100 ml Tetrahydrofuran bei 0 bis 5°C zugetropft. Nach 14stündigem Rühren bei Raumtemperatur wird das Reaktionsgemisch mit 50 g Eis auf 0°C abgekühlt, langsam mit 25 ml konz. Salzsäure versetzt und mit Äther extrahiert. Der Äther-Extrakt wird zweimal mit Wasser und zweimal mit gesättigter Kochsalzlösung gewaschen, über Natriumsulfat getrocknet, filtriert und eingeengt.

Das Produkt wird mit Essigester/Hexan (1 : 4) als Eluiermittel über Silikagel chromatographiert. Man erhält die Verbindung der Formel

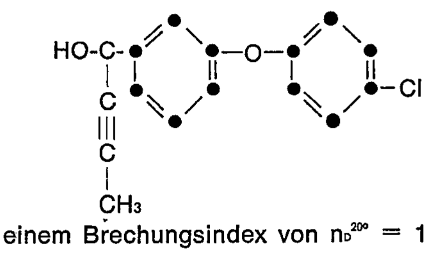

mit einem Brechungsindex von $n_D^{20°} = 1,5844$.

b) Herstellung con Prop-1-ynyl-3-phenoxybenzyl-2,2-dimethyl-3-(2',2'-dichlorvinyl)-cyclopropan-1-carboxylat

Zu einer eisgekühlten Lösung von 3,82 g 2,2-Dimethyl-3-(2',2'-dichlorvinyl)-cyclopropancarbonsäurechlorid und 1,8 ml Pyridin in 50 ml Toluol wird eine Lösung von 4,5 g α-Prop-1-ynyl-3-p-chlorphenoxybenzylalkohol in 20 ml Toluol zugetropft. Das Reaktionsgemisch wird 14 Stunden bei Raumtemperatur gerührt und dann mit Äther versetzt. Der Ätherextrakt wird einmal mit Wasser, einmal mit 2n-Salzsäure und dreimal mit gesättigter Kochsalzlösung gewaschen, anschliessend über Natriumsulfat getrocknet, filtriert und eingeengt. Das Produkt wird mit Äther/Hexan (1 : 3) als Eluiermittel über Silikagel chromatographiert.

Man erhält die Verbindung der Formel

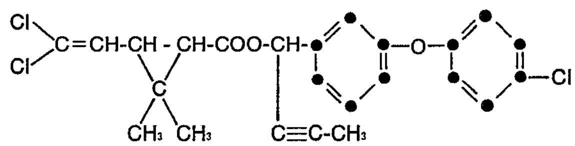

als ein diastereomeres Gemisch mit einem Brechungsindex von $n_D^{20°} = 1,5712$.

Auf analoge Weise werden auch folgende Verbindungen hergestellt:

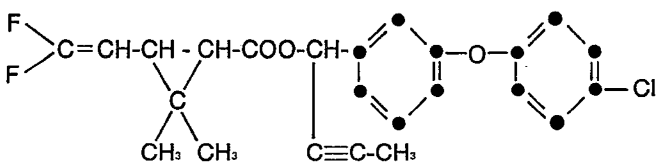

$n_D^{20°} = 1,5397$

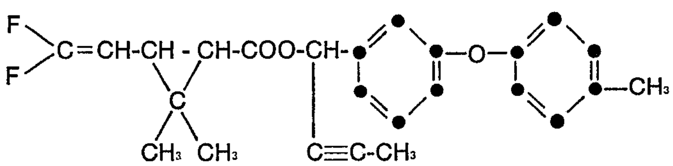

$n_D^{20°} = 1,5361$

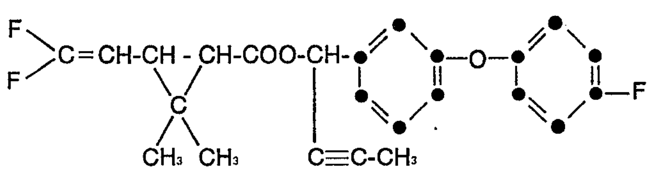

$n_D^{20°} = 1,5291$

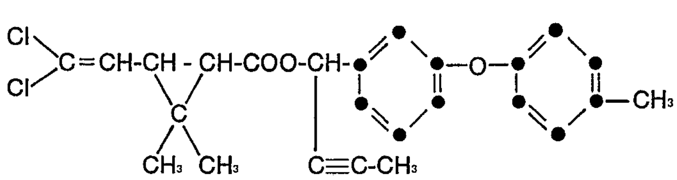

$n_D^{20°} = 1,5650$

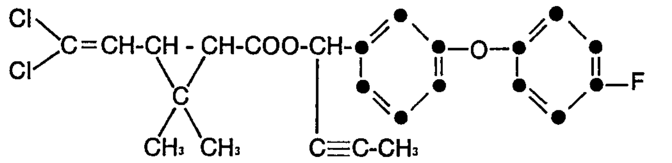

$n_D^{20^\circ} = 1{,}5581$

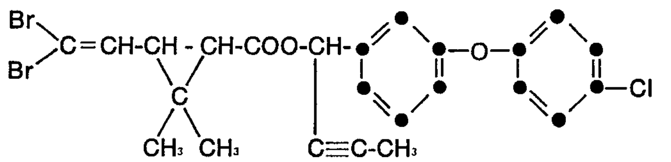

$n_D^{20^\circ} = 1{,}5397$

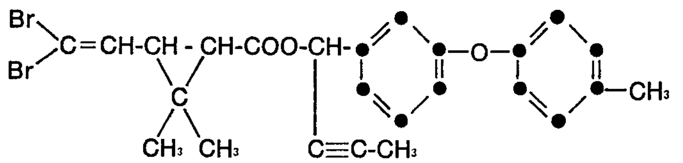

$n_D^{20^\circ} = 1{,}5832$

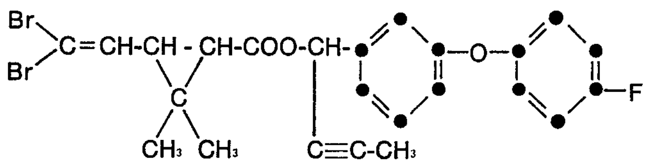

$n_D^{20^\circ} = 1{,}5740$

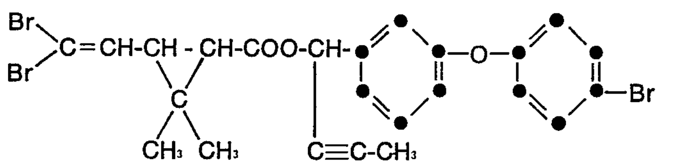

$n_D^{20^\circ} = 1{,}5981$

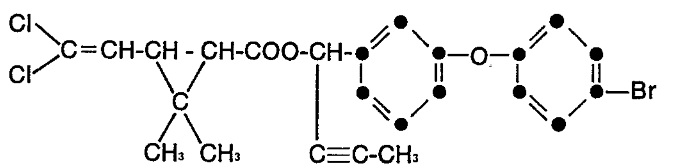

$n_D^{20^\circ} = 1{,}5819$

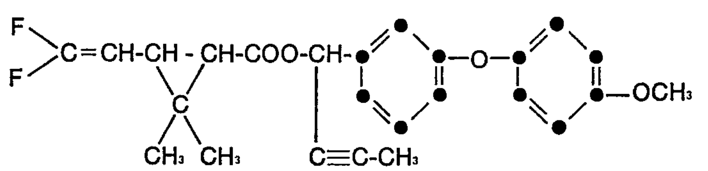

$n_D^{20^\circ} = 1{,}5400$

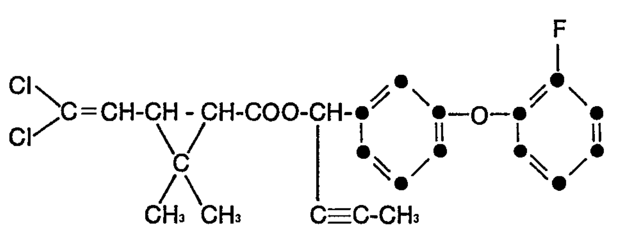

$n_D^{20^\circ} = 1{,}5420$

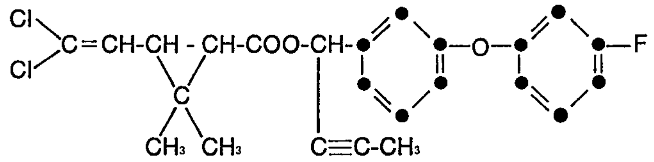

$n_D^{20°} = 1,5598$

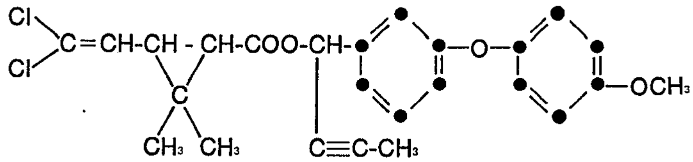

$n_D^{20°} = 1,5695$

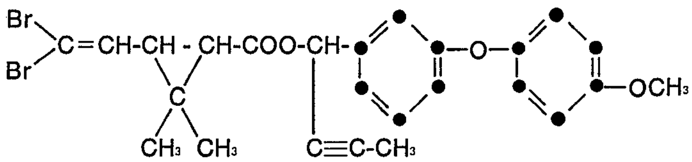

$n_D^{20°} = 1,5850$

Beispiel 2

a) Insektizide Frassgift-Wirkung

Baumwollpflanzen wurden mit einer 0,05%igen wässrigen Wirkstoffemulsion (erhalten aus einem 10%igen emulgierbaren Konzentrat) besprüht.

Nach dem Antrocknen des Belages wurden die Baumwollpflanzen je mit Spodoptera littoralis- und Heliothis virescens-Larven $L_3$ besetzt. Der Versuch wurde bei 24°C und 60% relativer Luftfeuchtigkeit durchgeführt.

Verbindungen gemäss Beispiel 1 zeigten im obigen Test eine gute insektizide Frassgift-Wirkung gegen Spodoptera- und Heliothis-Larven.

Beispiel 3

Akarazide Wirkung

Phaseolus vulgaris Pflanzen wurden 12 Stunden vor dem Test auf akarizide Wirkung mit einem infestierten Blattstück aus einer Massenzucht von Tetranychus urticae belegt. Die übergelaufenen beweglichen Stadien wurden aus einem Chromatographiezerstäuber mit den emulgierten Testpräparaten derart besprüht, dass kein Ablaufen der Spritzbrühe eintrat. Nach 2 und 7 Tagen wurden Larven, Adulte und Eier unter dem Binokular auf lebende und tote Individuen ausgewertet und das Ergebnis in Prozenten ausgedrückt. Während der «Haltezeit» standen die behandelten Pflanzen in Gewächshauskabinen bei 25°C.

Verbindungen gemäss Beispiel 1 wirkten im obigen Test gegen Adulte, Larven und Eier von Tetranychus urticae.

Beispiel 4

a) Rhipicephalus bursa

Je 5 adulte Zecken bzw. 50 Zeckenlarven wurden in ein Glasröhrchen gezählt und für 1 bis 2 Minuten in 2 ml einer wässrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

b) Boophilus microplus (Larven)

Mit einer analogen Verdünnungsreihe wie beim Test a) wurden mit je 20 sensiblen resp. OP-resistenten Larven Versuche durchgeführt. (Die Resistenz bezieht sich auf die Verträglichkeit von Diazinon.) Verbindungen gemäss Beispiel 1 wirkten in diesen Tests gegen Adulte und Larven von Rhipicephalus bursa und sensible resp. OP-resistente Larven von Boophilus microplus.

**Patentansprüche für die Vertragsstaaten: BE, CH, DE, FR, GB, IT, NL**

1. Ein Cyclopropancarbonsäureester der Formel

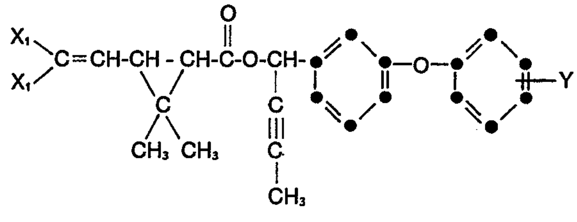

worin

$X_1$ Fluor, Chlor oder Brom und

Y Methoxy, Methyl, Fluor, Chlor oder Brom bedeuten.

2. Eine Verbindung gemäss Anspruch 1, worin $X_1$ Fluor, Chlor oder Brom und Y p-Methoxy, p-Methyl, p-Fluor, p-Chlor oder p-Brom bedeuten.

3. Die Verbindung gemäss Anspruch 2 der Formel

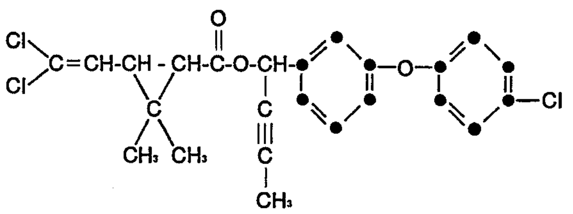

4. Die Verbindung gemäss Anspruch 2 der Formel

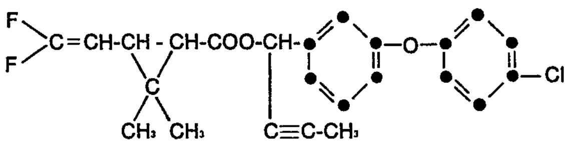

5. Verfahren zur Herstellung einer Verbindung gemäss Anspruch 1, dadurch gekennzeichnet, dass man eine Verbindung der Formel

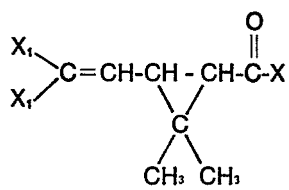

in Gegenwart eines säurebindenden Mittels mit einer Verbindung der Formel

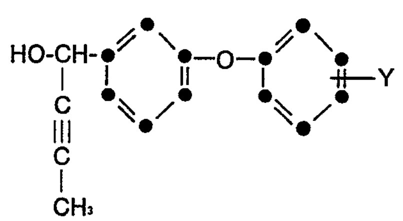

umsetzt, worin $X_1$ und Y die im Anspruch 1 angegebene Bedeutung haben und X für ein Halogenatom steht.

6. Ein Schädlingsbekämpfungsmittel, welches als aktive Komponente eine Verbindung gemäss Anspruch 1 und geeignete Träger- und/oder andere Zuschlagstoffe enthält.

7. Verwendung einer Verbindung gemäss Anspruch 1 zur Bekämpfung von verschiedenartigen tierischen und pflanzlichen Schädlingen.

8. Verwendung gemäss Anspruch 7 zur Bekämpfung von Insekten und Vertretern der Ordnung Akarina.

**Patentanspruch für den Vertragsstaat: AT**

Ein Mittel zur Bekämpfung von Insekten und Vertretern der Ordnung Akarina, dadurch gekennzeichnet, dass es neben 5 bis 99,9% eines geeigneten Träger- und/oder andern Zuschlagstoffes als aktive Komponente 0,1 bis 95% einer Verbindung der allgemeinen Formel

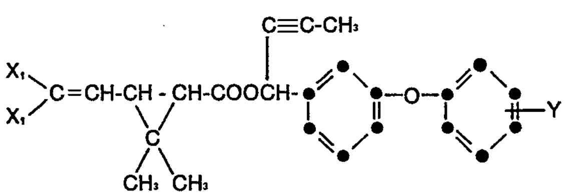

enthält, worin
$X_1$ Fluor, Chlor oder Brom und
Y Methoxy, Methyl, Fluor, Chlor oder Brom bedeuten.

**Claims for the Contracting States: BE, CH, DE, FR, GB, IT, NL**

1. A cyclopropanecarboxylic acid ester of the formula

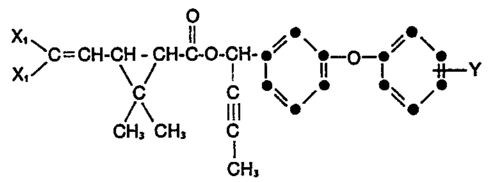

(I)

in which
$X_1$ is fluorine, chlorine or bromine, and
Y is methoxy, methyl, fluorine, chlorine or bromine.

2. A compound according to Claim 1, wherein $X_1$ is fluorine, chlorine or bromine, and Y is p-methoxy, p-methyl, p-fluorine, p-chlorine or p-bromine.

3. The compound according to Claim 2 of the formula

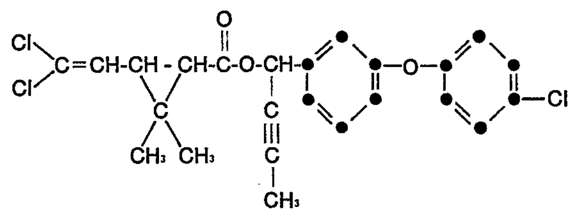

4. The compound according to Claim 2 of the formula

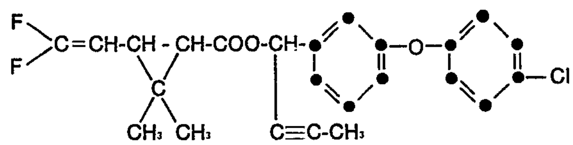

5. A process for producing a compound according to Claim 1, which process comprises reacting a compound of the formula

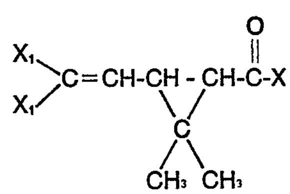

in the presence of an acid-binding agent, with a compound of the formula

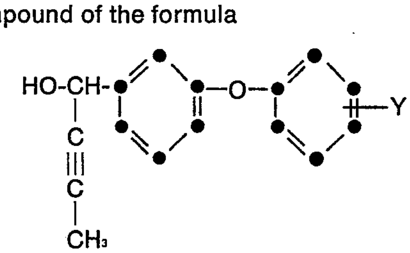

wherein $X_1$ and Y have the meanings given in Claim 1, and X is a halogen atom.

6. A pesticidal composition which comprises a compound according to Claim 1 as active ingredient, and suitable carriers and/or other additives.

7. Use of a compound according to Claim 1 for combating various animal and plant pests.

8. Use according to Claim 7 for combating insects, and representatives of the order Acarina.

**Claim for the Contracting State: AT**

A composition for combating insects, and members of the order Acarina, characterised in that it contains as active ingredient 0.1 to 95% of a compound of the general formula

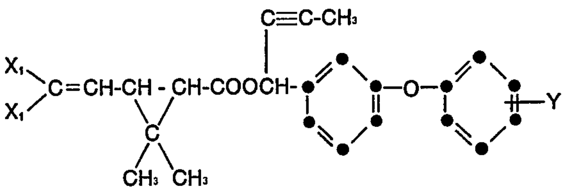

wherein
$X_1$ is fluorine, chlorine or bromine, and
Y is methoxy, methyl, fluorine, chlorine or bromine, and 5 to 99.9% of a suitable carrier and/or other additive.

**Revendications pour les Etats contractants: BE, CH, DE, FR, GB, IT, NL**

1. Ester d'acide cyclopropanecarboxylique de formule

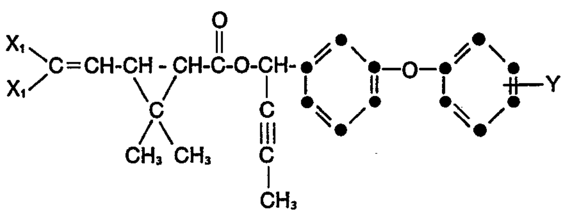

où
$X_1$ représente un fluor, un chlore ou un brome et
Y représente un méthoxy, un méthyle, un fluor, un chlore ou un brome.

2. Composé selon la revendication 1, où $X_1$ représente un fluor, un chlore ou un brome et où Y représente un p-méthoxy, p-méthyle, p-fluor, p-chlore ou p-brome.

3. Composé selon la revendication 2 de formule

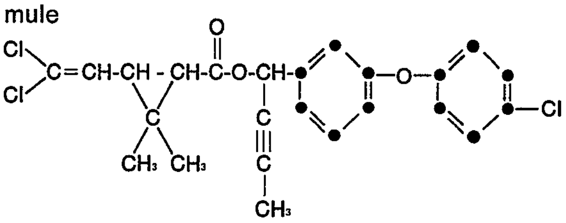

4. Composé selon la revendication 2 de formule

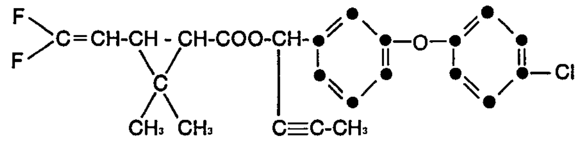

5. Procédé de préparation d'un composé selon la revendication 1, caractérisé en ce qu'on fait réagir un composé de formule

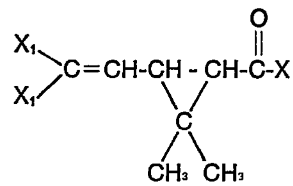

en présence d'un liant acide avec un composé de formule

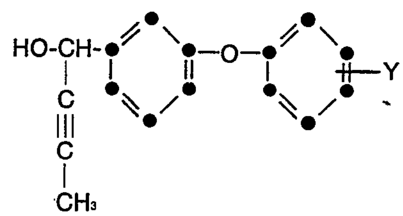

où $X_1$ et Y ont la signification donnée dans la revendication 1 et où X représente un atome d'halogène.

6. Agent de lutte antiparasitaire contenant comme composant actif un composé selon la revendication 1 et des supports et/ou autres additifs appropriés.

7. Application d'un composé selon la revendication 1 à la lutte contre différentes espèces de parasites animaux et végétaux.

8. Application selon la revendication 7 à la lutte contre les insectes et les représentants de l'ordre des Acariens.

**Revendication pour l'Etat contractant: AT**

Agent de lutte contre les insectes et les représentants de l'ordre des Acariens, caractérisé en ce qu'il contient, outre de 5 à 99,9% d'un support et/ou autre additif approprié, comme composant actif de 0,1 à 95% d'un composé de formule générale

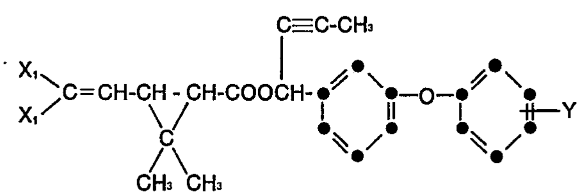

où
$X_1$ représente un fluor, un chlore ou un brome et
Y représente un méthoxy, un méthyle, un fluor, un chlore ou un brome.